# EUROPEAN PATENT SPECIFICATION

(11) **EP 1 805 307 B1**
(45) Date of publication and mention of the grant of the patent: **14.01.2009**
(21) Application number: 05795379.6
(22) Date of filing: 20.10.2005
(51) Int. Cl.: C12N 15/11, C12N 15/88

(54) **PROTECTION OF BIOLOGICALLY ACTIVE MOLECULES USING AMPHIPHILES**
SCHUTZ VON BIOLOGISCH AKTIVEN MOLEKÜLEN MITTELS AMPHIPHILEN
PROTECTION DE MOLÉCULES BIOLOGIQUEMENT ACTIVES AU MOYEN D'AMPHIPHILES

(30) Priority: 21.10.2004 NL 1027311; 04.11.2004 NL 1027417; 10.11.2004 NL 1027479
(43) Date of publication of application: 11.07.2007
(73) Proprietor: Synvolux IP B.V., 9713 GX Groningen (NL)
(72) Inventor: RUITERS, Marcel, Herman, Josef, NL-9728 WN Groningen (NL)
(74) Representative: Kupecz, Arpad
(86) International application number: PCT/NL2005/000752
(87) International publication number: WO 2006/043809

(56) References cited:
- EP-A- 0 755 924
- REJMAN J ET AL: "Characterization and transfection properties of lipoplexes stabilized with novel exchangeable polyethylene glycol-lipid conjugates" BIOCHIMICA ET BIOPHYSICA ACTA. BIOMEMBRANES, AMSTERDAM, NL, vol. 1660, no. 1-2, 28 January 2004 (2004-01-28), pages 41-52, XP004486900 ISSN: 0005-2736
- ZUHORN I S ET AL: "Interference of serum with lipoplex-cell interaction: modulation of intracellular processing" BIOCHIMICA ET BIOPHYSICA ACTA. BIOMEMBRANES, AMSTERDAM, NL, vol. 1560, no. 1-2, 18 February 2002 (2002-02-18), pages 25-36, XP004342702 ISSN: 0005-2736
- OBERLE VOLKER ET AL: "Lipoplex formation under equilibrium conditions reveals a three-step mechanism" BIOPHYSICAL JOURNAL, vol. 79, no. 3, September 2000 (2000-09), pages 1447-1454, XP002364029 ISSN: 0006-3495

## Description

The present invention relates to a process to protect a biologically active compound against the effects of for instance enzymes, chemicals, oxygen, radicals or sunlight. Further the invention relates to a vehicle to protect a biological active compound against the effects of for instance enzymes, chemicals, oxygen, radicals or sunlight. The invention also relates to the application of a SAINT-molecule to protect a biological active compound against the effects of for instance enzymes, chemicals, oxygen, radicals or sunlight.

In general, biologically active compounds, such as siRNA, RNA, DNA, proteins and peptides are sensitive for the effects (e.g. degradation) of for instance enzymes, chemicals, oxygen, radicals or sunlight. For example, in many cases biologically active compounds, such as siRNA, RNA, DNA, oligonucleotides or derivates thereof, proteins and peptides can only be transported as dry matter, or as a stabilized, glycerol or DMSO containing solution. The solutions to be transported or dry matters are kept on dry ice, or are cooled in another way. Consequently, the transportation of these compounds is very expensive.

Another disadvantage of transporting biologically active compounds as dry matter is the fact that during the dissolution of the biologically active compound a conformational change may occur. As a result, not all biologically active matter will regain its original function.

Also the transport of the biologically active compound in a (buffered) glycerol (or DMSO) containing solution has disadvantages. Glycerol (or DMSO) has shown to have a negative effect on the activity of the biologically active compound when the biologically active compound is diluted before application.

The solution found in the state of the art to circumvent these disadvantages is to replace the glycerol (or DMSO) by trehalose. Trehalose is a sugar. Although in theory no conformational change occurs when dissolving the biologically active compound in trehalose, there are some other disadvantages when using trehalose. A particular disadvantage is that trehalose has an adverse effect on the desired enzymatic activity of the proteins. In the state of the art trehalose is not used to package DNA, RNA, siRNA en oligonucleotides or derivatives thereof. After complexation with trehalose (and other sugars) DNA,RNA etc, are laboriously to be dissolved, as they can also be characterized as sugars.

REJMAN J ET AL: "Characterization and transfection properties of lipoplexes stabilized with novel exchangeable polyethylene glycol-lipid conjugates" BIOCHIMICA ET BIOPHYSICA ACTA. BIOMEMBRANES, AMSTERDAM, NL, vol. 1660, no. 1-2,28 January 2004 (2004-01-28), pages 41-52, XP004486900 ISSN: 0005-2736 discloses characterization and transfection properties of lipoplexes stabilized with novel exchangeable polyethylene glycol-lipid conjugates.

ZUHORN I S ET AL: "Interference of serum with lipoplex-cell interaction: modulation of intracellular processing" BIOCHIMICA ET BIOPHYSICA ACTA. BIOMEMBRANES, AMSTERDAM, NL, vol. 1560, no. 1-2, 18 February 2002 (2002-02-18), pages 25-36, XP004342702 ISSN: 0005-2736 discloses interference of serum with lipoplex-cell interaction: modulation of intracellular processing.

The present invention aims at solving the disadvantages mentioned above.

In particular, the invention aims to provide for a process to protect a biologically active compound against the effects of for instance enzymes, chemicals, oxygen, radicals or sunlight.

The invention also aims to provide for a vehicle to protect a biologically active compound against the effects of enzymes, chemicals, oxygen, radicals or sunlight whereby the disadvantages mentioned above can be circumvented.

The word "transport" is understood to mean both the packaging of the biologically active compound in a transportation container, and the subsequent conveyance of this container by means of transportation means, such as carriers, etc., as well as the transport of individual biologically active molecules, which are each separately enwrapped by the protecting compounds.

To meet at least one of the aforementioned aims, the present invention provides for a process as mentioned in the preamble, which process is characterised by the measures according to claim 1. Herewith the advantage is reached that the biologically active compound has become completely protected from effects of enzymes, chemicals, oxygen, radicals or sunlight. Furthermore, completely no or no functional inhibitory conformational changes of the biological compound occur.

Furthermore the invention provides for a vehicle as mentioned in the pre-amble, characterized by the measures as described in claim 4. The vehicle of the present invention provides a very reliable and simple solution for transporting a biologically active compound in such a way that the biologically active compound will not become damaged, for instance by the effect of enzymes, chemicals, oxygen, radicals or sunlight, or a conformational change occurs.

According to a further aspect of the invention an application is provided in which a SAINT-molecule protects a biologically active compound against the effect of for instance enzymes, chemicals, oxygen, radicals or sunlight, which is characterized in that the biologically active compound is contacted with the biologically active compound, causing the biologically active compounds to interact with the SAINT-molecule.

By means of the invention, the biologically active compound, which can be chosen from, for instance, siRNA, RNA, DNA, oligonucleotides or derivatives thereof, proteins and peptides, is actually enwrapped by one ore more SAINT-molecules. The SAINT molecules may all be of the same kind, but it is also possible that a mixture of different SAINT-molecules is applied, which can be connected to the biological active compound. This interaction is a hydrogen-bond.

It has been shown that the vehicle of the present invention, consisting of the biologically active compound and the SAINT-molecule or SAINT-molecules enwrapping it, can be kept without occurring a separation of the biologically active compound and the SAINT-molecules. Nevertheless, it has also been shown that, when the biologically active compound has to fulfil its function, it is not suffering any functional hindrance from the SAINT-molecules present. Therefore the biologically active compounds can freely operate and can easily be applied in basically any biological assay. A remarkably great advantage of the present invention is that no separation of the SAINT-molecule(s) from the biological active compound is needed, because the saint-molecule(s) have no inhibitory effect in the buffer. The molecule will be diluted to such a great extent, that no inhibitory effect will be shown. Furthermore it is biologically degradable, without forming any toxic compounds.

### EXAMPLE

One of the most sensitive and at this moment frequently used biologically active compounds is siRNA. SiRNA, when directed to a specific gene, is able to silence the gene-expression by inhibition of the mRNA translation.

SiRNA needs to be stored as dried powder and, after dissolution, aliquots are stored at -20°C. Regardless of aliquots being prepared, the reproducibility of (the results obtained over time using) such aliquots is weak. The delivery of siRNA to cells has been described in patents EP-0755924 and US 5,853,694.

To illustrated the present invention we here show data concerning the preservation of siRNA by the use of SAINT-molecules.

### Method:

25 micrograms of siRNA were complexed with 0,5 ml of SAINT-MIX. Per transfection 20 µl of this (preservation)-complex is used. Transfection was performed as described in detail in EP-0755924.

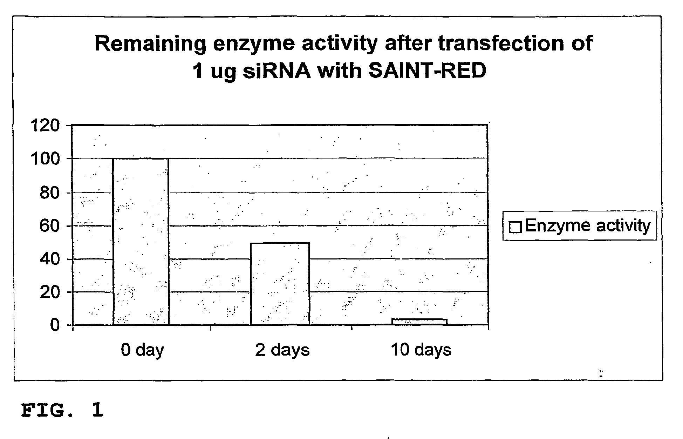

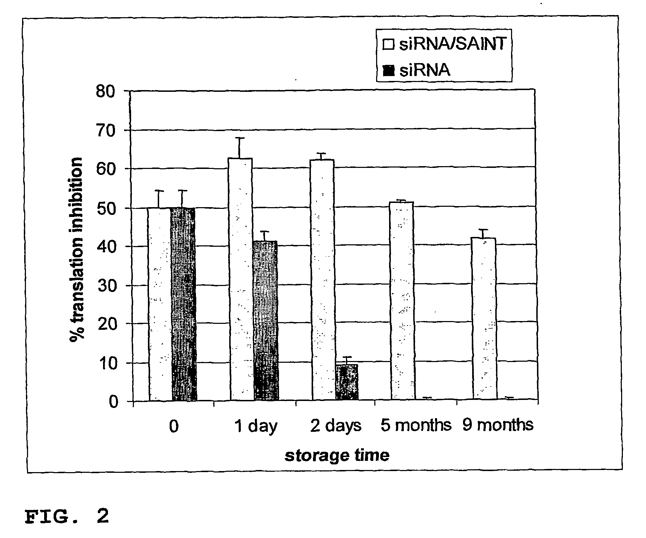

The efficacy of the siRNA-transfection is validated by measuring the enzyme activity of the silenced gene according to a standard enzyme assay. Lifetime of the enzyme is approximately 5 days. Therefore, after one single transfection with 1 µg siRNA, about 50% enzyme activity can be measured after 48 hours and almost no enzyme activity can be measured after 10 days, as shown in the figure 1 below. 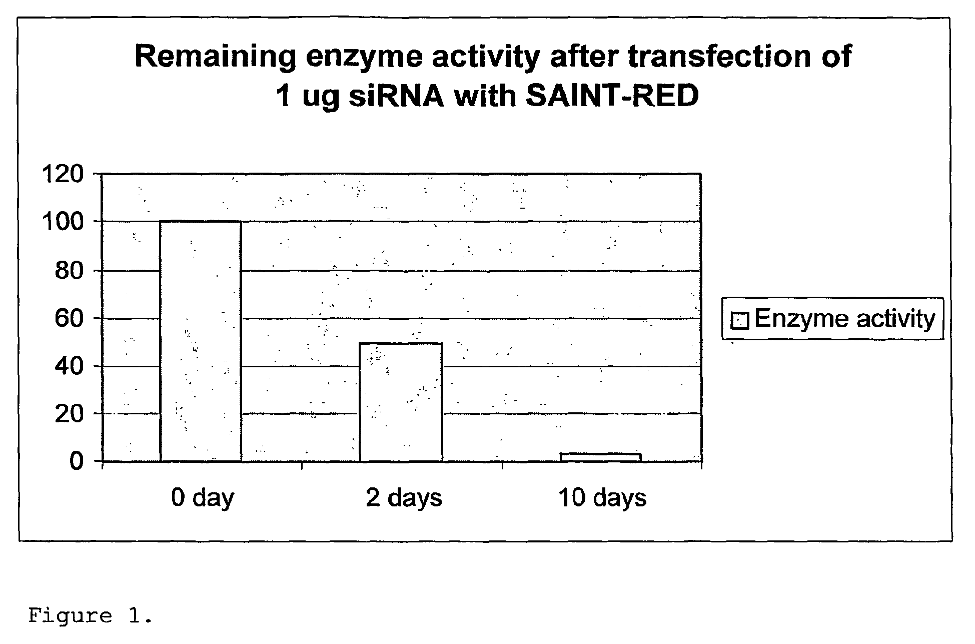 Over a 9 month-period, we compared the siRNA stored at -20°C degrees versus the siRNA/SAINT-RED preservation complex. Enzyme activity was measured 48 hours after transfection. Results have been depicted in Figure 2.

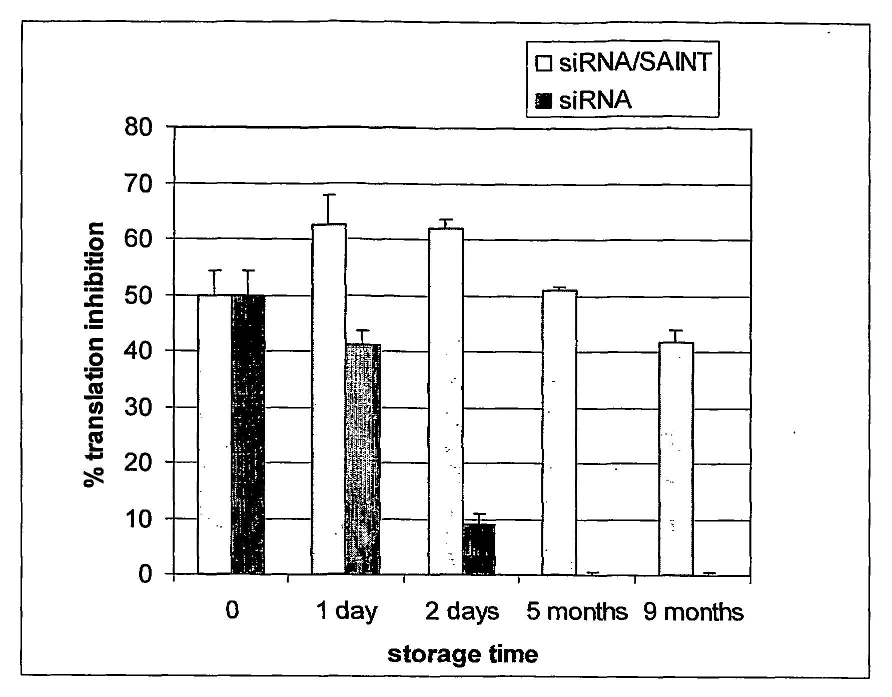

### Figure 2.

The figure clearly shows that siRNA complexed with SAINT-RED is active over a 9 month- period while the aliquoted siRNA has lost almost all its activity within 2 days. Moreover the results show that the siRNA/SAINT prevention complex is even more active, up to 5 months, when compared with the freshly prepared siRNA/SAINT complex.

This indicates the strong protective character of SAINT-molecules towards biologically active compounds.

## Claims

1. A process for the protection of a biologically active compound over a period of days, weeks and months **characterised in that** it contains the step of bringing the biologically active compound in contact with a SAINT-molecule.

2. The process according to claim 1, **characterised in that** this step comprises the contacting of a biologically active compound with a mixture of SAINT-molecules.

3. The process according to claims 1 or 2, **characterised in that** the biologically active compound is bound to at least one SAINT-molecule by means of electrostatic interaction.

4. A process to preserve a biologically active over a period of days, weeks and months, **characterised in that** it comprises the step of combining the biologically active compound with a SAINT-molecule or a mixture thereof.

5. The application of a SAINT-molecule for the protection of a biologically active compound over a period of days, weeks and months, whereby the biologically active compound is brought in contact with a SAINT-molecule in such a way that the biologically active compound interacts with the SAINT-molecule through electrostatic interaction.

## Patentansprüche

1. Verfahren zum Schutz einer biologisch aktiven Verbindung über einen Zeitraum von Tagen, Wochen und Monaten, **dadurch gekennzeichnet, dass** es den Schritt beinhaltet, die biologisch aktive Verbindung in Kontakt mit einem SAINT-Molekül zu bringen.

2. Verfahren nach Anspruch 1, **dadurch gekennzeichnet, dass** dieser Schritt beinhaltet, eine biologisch aktive Verbindung mit einem Gemisch aus SAINT-Molekülen in Kontakt zu bringen.

3. Verfahren nach Anspruch 1 oder 2, **dadurch gekennzeichnet, dass** die biologisch aktive Verbindung an mindestens ein SAINT-Molekül mittels elektrostatischer Wechselwirkung gebunden ist.

4. Verfahren zur Konservierung einer biologisch aktiven Verbindung über einen Zeitraum von Tagen, Wochen und Monaten, **dadurch gekennzeichnet, das** es den Schritt aufweist, die biologisch aktive Verbindung mit einem SAINT-Molekül oder einem Gemisch aus diesem zu kombinieren.

5. Verwendung eines SAINT-Moleküls für den Schutz einer biologisch aktiven Verbindung über einen Zeitraum von Tagen, Wochen und Monaten, bei der die biologisch aktive Verbindung in Kontakt mit einem SAINT-Molekül gebracht wird, derart, dass die biologisch aktive Verbindung mit dem SAINT-Molekül mittels elektrostatischer Wechselwirkung interagiert.

## Revendications

1. Procédé de protection d'un composé biologiquement actif pendant une période de plusieurs jours, semaines et mois, **caractérisé en ce qu'**il contient l'étape consistant à mettre le composé biologiquement actif en contact avec une molécule SAINT.

2. Procédé selon la revendication 1, **caractérisé en ce que** cette étape comprend la mise en contact d'un composé biologiquement actif avec un mélange de molécules SAINT.

3. Procédé selon la revendication 1 ou 2, **caractérisé en ce que** le composé biologiquement actif est lié à au moins une molécule SAINT au moyen d'une interaction électrostatique.

4. Procédé de préservation d'un composé biologiquement actif, pendant une période de plusieurs jours, semaines et mois, **caractérisé en ce qu'**il comprend l'étape consistant à combiner le composé biologiquement actif avec une molécule SAINT ou un mélange de ce type de molécules.

5. Application d'une molécule SAINT pour la protection d'un composé biologiquement actif pendant une période de plusieurs jours, semaines et mois, ce par quoi le composé biologiquement actif est mis en contact avec une molécule SAINT de façon à ce que le composé biologiquement actif interagisse avec la molécule SAINT via une interaction électrostatique.
